# EUROPÄISCHE PATENTSCHRIFT

(11) **EP 2 790 789 B1**
(45) Veröffentlichungstag und Bekanntmachung des Hinweises auf die Patenterteilung: **11.01.2017**
(21) Anmeldenummer: 12790834.1
(22) Anmeldetag: 09.11.2012
(51) Int. Cl.: A61Q 5/06, A61K 8/81, A61K 8/02, D21B 1/32, A45D 7/04, A45D 7/00

(54) **HAARKOSMETIKA ENTHALTEND POLYMERKOMBINATION**
HAIR COSMETICS COMPRISING POLYMER COMBINATION
PRODUITS COSMÉTIQUES CAPILLAIRES COMPRENANT DES POLYMÈRES

(30) Priorität: 16.12.2011 DE 102011088841
(43) Veröffentlichungstag der Anmeldung: 22.10.2014
(73) Patentinhaber: Henkel AG & Co. KGaA, 40589 Düsseldorf (DE)
(72) Erfinder: KNAPPE, Thorsten, 22869 Schenefeld (DE); HENSCHEL, Anna, 21423 Winsen (DE); LANGE, Bibiane, 24641 Sievershütte (DE)
(86) Internationale Anmeldenummer: PCT/EP2012/072237
(87) Internationale Veröffentlichungsnummer: WO 2013/087312

(56) Entgegenhaltungen:
- EP-A2- 1 360 955
- WO-A1-2007/073857
- DE-A1-102009 002 267
- DATABASE GNPD [Online] MINTEL; Mai 2011 (2011-05), "Smoother Sleeker Blowdry Hybrid", XP002711633, Database accession no. 1545882
- DATABASE GNPD [Online] MINTEL; August 2006 (2006-08), "Styling Whip", XP002711634, Database accession no. 576924
- anonymous: "Der trnd Projektblog: Hier gibt's die aktuellen Projektnews und Diskussionen rund um das trnd-Projekt mit dem got2b POWDER'ful Styling Powder.", , 16. Februar 2011 (2011-02-16), XP002711635, Gefunden im Internet: URL:http://got2b-powderful.trnd.com/2011/0 2/16/unsere-tipps-fur-den-umgang-mit-got2b -powderful/ [gefunden am 2012-08-22]
- DATABASE GNPD [Online] MINTEL 01 April 2011 SCHWARZKOPF & HENKEL: 'Volume Styling Powder' Database accession no. 1524156

## Beschreibung

Die vorliegende Erfindung betrifft das technische Gebiet der Pflege keratinhaltiger Fasern, insbesondere der temporären Umformung keratinhaltiger Fasern, wie z.B. der temporären Haarumformung.

Stylingmittel zur Verformung keratinhaltiger Fasern sind lange bekannt und finden in verschiedener Ausgestaltung Einsatz wie beispielsweise zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Bekannte Formen temporärer Stylingmittel lassen sich oftmals nicht mit zufriedenstellender Genauigkeit dosieren. So lassen sich etwa Haargele, Haarcremes und Haarwachse nur noch schwierig verteilen, sobald sie einmal auf das Haar aufgebracht sind. Sobald der Kamm oder die Hände, auf die das Stylingmittel aufgebracht wurde, mit den ersten Haarpartien in Kontakt kommen, werden vergleichsweise große Mengen an Stylingmittel an das Haar abgegeben. In Haarpartien, die erst später mit dem Kamm oder den Händen erreicht werden, wird hingegen vergleichsweise wenig Stylingmittel eingearbeitet. Dies hat zur Folge, dass der Anwender entweder von Anfang an eine große Menge Stylingmittel aufbringen muss, so dass auch die zuletzt erreichten Haarpartien ausreichend Stylingmittel erhalten, oder gezwungen ist, das Stylingmittel in mehreren Schritten aufzubringen, wobei jeweils andere Haarpartien behandelt werden. Haarsprays lassen sich gleichmäßiger auf das Haar verteilen. Da der Verwender aber keine Möglichkeit hat, die Gesamtmenge an aufgebrachtem Stylingmittel visuell zu erfassen, besteht die Gefahr, dass mehr Stylingmittel auf das Haar aufgebracht wird, als eigentlich erforderlich wäre.

Pulverförmige Kosmetika sind bekannt und werden etwa im Bereich der Hautbehandlung bereits seit langem eingesetzt. Typische Beispiele sind etwa Make-up Puder oder Lidschatten. Um die pulverförmige Konsistenz zu erzielen, ist der Einsatz eines pulverförmigen Trägermaterials erforderlich. Als geeignetes Trägermaterial kann etwa ein Metalloxid, wie z.B. Siliziumdioxid verwendet werden. Der Einsatz von Metalloxiden als Trägermaterial führt jedoch meist zu einer Ablagerung des Metalloxids auf der keratinhaltigen Faser. Die mit einem solchen pulverförmigen Kosmetikum behandelte keratinhaltige Faser verliert durch diese Ablagerung ihren natürlichen Glanz und wirkt matt. Des weiteren kennt der Fachmann pulverförmige oder granulierte Kosmetika beispielsweise aus dem Bereich der Frisörprodukte. Der Frisör stellt aus dem Pulver oder Granulat durch Zugabe eines Überschusses Wasser eine definierte Menge des Haarkosmetikums her, welches im Anschluss daran als Creme, Gel oder Lotion auf dem Haar angewendet wird. Aus dem Bereich der temporären Umformung keratinhaltiger Fasern kennt der Fachmann lösmittelhaltige Pulver in Form einer powder-to-liquid Formulierung. Die Druckschrift WO 2007/051511 A1 offenbart die Verwendung einer pulverförmigen Zusammensetzung, enthaltend 50 bis 95 Gew.-% eines wässrigen Lösungsmittels, hydrophobiertes Siliziumdioxidpulver und mindestens im wässrigen Lösemittel befindliches filmbildendes und/oder festigendes Polymer zur temporären Verformung keratinischer Fasern.

Die Druckschrift WO 2010/054980 A1 offenbart die Verwendung einer pulverförmigen Zusammensetzung mit Kern-Hülle-Teilchen zur temporären Verformung keratinischer Fasern, wobei die Hülle die Partikel mindestens eines hydrophobierten Metalloxidpulvers enthält und der Kern eine flüssige, wässrige Phase umfasst. Diese pulverförmigen Kern-Hülle-Teilchen umfassen mindestens ein filmbildendes und/oder festigendes Polymer in Form von Partikeln.

In der Druckschrift WO-A1-2011/076518 werden pulverförmige Zusammensetzungen beschrieben, die sich in eine Flüssigkeit umwandeln lassen und die einen auf einem Träger sorbierten Wirkstoff umfassen.

Die pulverförmigen Haarkosmetika des Standes der Technik liefern mittlerweile zwar einen für die Haarumformung akzeptablen Halt. Allerdings ist das mit diesen Mitteln erzielte Ergebnis bezüglich der Parameter Glanz und Volumen sowie der Elastizität des Halts verbesserungswürdig. Ferner sind die bekannten pulverförmigen Zusammensetzungen nicht mit allen kosmetischen Rohstoffen kompatibel und weisen eine beschränkte Lagerstabilität auf.

Aufgabe der vorliegenden Erfindung war es daher, ein Haarbehandlungsmittel zur temporären Formgebung zur Verfügung zu stellen, das genau und einfach dosiert werden kann, sowie auf der Faser einen verbesserten Glanz, Volumen sowie einen elastischen Halt bewirkt. Die Haltbarkeit des Stylingergebnisses soll dabei nicht beeinträchtigt werden. Wenn das Haarbehandlungsmittel als Pulver vorliegt, soll es bei Kontakt mit wenig Wasser nicht verklumpen, sondern sich gut auf den Fasern verteilen lassen.

Es wurde gefunden, dass die Lehre des Standes der Technik durch eine nachfolgend beschriebene Zusammensetzung, in Pulverform, verbessert wird. Zusammensetzungen mit nachfolgendem Anforderungsprofil zur Anwendung auf der Faser im Rahmen einer Umformung keratinhaltiger Fasern sind neu.

Ein erster Gegenstand der vorliegenden Erfindung sind pulverförmige kosmetische Mittel, enthaltend als Komponente (a) mindestens ein Polymer, umfassend mindestens eine Struktureinheit der Formel (I) und mindestens eine Struktureinheit der Formel (II), 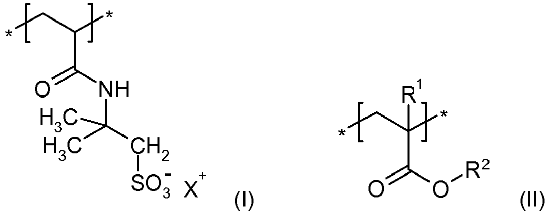 worin
- X⁺: für ein physiologisch verträgliches Kation (insbesondere Na⁺) steht,
- R¹: für ein Wasserstoffatom oder eine Methylgruppe steht und
- R²: für eine (C₂ bis C₆)-Hydroxyalkylgruppe steht,
und als Komponente (b) mindestens ein festigendes Polymer.

Unter keratinhaltigen Fasern sind dabei erfindungsgemäß Pelze, Wolle, Federn und insbesondere menschliche Haare zu verstehen.

Unter Polymeren werden erfindungsgemäß Verbindungen verstanden, die aus einer Vielzahl von Molekülen aufgebaut sind, in denen eine Art oder mehrere Arten von Atomen oder Atomgruppierungen (sogenannte konstitutive Einheiten, Grundbausteine oder Wiederholungseinheiten) wiederholt aneinander gereiht sind und die ein Molekulargewicht von wenigstens 10000 g/mol besitzen. Die Polymere werden durch Polyreaktion erhalten, wobei letztere künstlich (d.h. synthetisch) oder natürlich erfolgen kann.

Die Struktureinheit der Formel (I) geht auf 2-Methyl-2[(1-oxo-2-propenyl)amino]-1-propansulfonsäure (AMPS) zurück, die teilweise oder vollständig neutralisiert vorliegen kann. Üblicherweise sind als Kationen Na⁺ und NH⁴⁺ bevorzugt.

Steht gemäß obiger Formel (II) der Rest R² für eine 2-Hydroxyethylgruppe, sind die resultierenden Polymere der Komponente (a) der erfindungsgemäßen Mittels am bevorzugtesten.

Bevorzugte Polymere der Komponente (a) des erfindungsgemäßen Mittels werden mit der INCI-Bezeichnung Hydroxyethylacrylate / Sodium Acryloyldimethyl Taurate Copolymer beispielsweise von der Firma Seppic unter dem Handelsnamen Sepinov^{®} EMT 10 in Pulverform vertrieben. Unabhängig davon, ob die erfindungsgemäßen Mittel ein oder mehrere Polymere der erfindungsgemäßen Komponente (a) enthalten, ist es bevorzugt, diese Polymere innerhalb bestimmter Mengenbereiche einzusetzen. Hierbei sind erfindungsgemäße Mittel bevorzugt, die die besagten Polymere der Komponente (a) in einer Menge von zwischen 5 und 95 Gew.-%, insbesondere von 0,1 bis 50 Gew.-%, besonders bevorzugt von 5,0 bis 40,0 Gew.-% und ganz besonders bevorzugt von 10,0 bis 30,0 Gew.-%, jeweils bezogen auf das Gewicht des Mittels, enthalten.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Unter filmbildenden Verbindungen bzw. filmbildenden Polymeren sind solche Verbindungen/Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden.

Unter filmbildenden Verbindungen bzw. filmbildenden Polymeren werden weiterhin solche Verbindungen bzw. Polymere verstanden, die bei Anwendung in 0,01 bis 20 Gew.-%-iger wässriger, alkoholischer oder wässrigalkoholischer Lösung in der Lage sind, auf dem Haar einen transparenten Film abzuscheiden.

Die festigenden Polymere sind bevorzugt in einer Menge von 2,0 Gew.-% bis 95 Gew.-%, insbesondere von 5,0 Gew.-% bis 95,0 Gew.-%, ganz besonders bevorzugt von 7,5 bis 95,0 Gew.-%, jeweils bezogen auf das Gesamtgewicht des kosmetischen Mittels, enthalten Bevorzugte kosmetische Mittel enthalten als festigendes Polymer der Komponente (b) mindestens ein festigendes Polymer ausgewählt wird aus Polymeren umfassend mindestens eine Lactam-Struktureinheit. Diese Polymere können wiederum bevorzugt nichtionisch oder kationisch sein.

Als Lactam-Struktureinheit sind insbesondere die Pyrrolidon-1-yl-Gruppe und die Caprolactam-1-yl-Gruppe zu nennen, wobei Pyrrolidon-1-yl-Gruppen als Lactam-Struktureinheit besonders bevorzugt sind.

Im Rahmen einer besonders bevorzugten Ausführungsform enthalten die erfindungsgemäßen pulverförmigen kosmetischen Mittel als filmbildendes Polymer der Komponente (b) mindestens ein festigendes nichtionisches Polymer. Dieses weist wiederum bevorzugt mindestens eine Lactam-Struktureinheit auf.

Die festigenden nichtionischen Polymere sind in dem erfindungsgemäßen Mittel bevorzugt in einer Menge von 2,0 Gew.-% bis 95 Gew.-%, insbesondere von 5,0 Gew.-% bis 95,0 Gew.-%, ganz besonders bevorzugt von 7,5 bis 95,0 Gew.-%, jeweils bezogen auf das Gesamtgewicht des erfindungsgemäßen Mittels, enthalten.

Die festigenden nichtionischen Polymere werden wiederum bevorzugt ausgewählt aus mindestens einem Polymer der Gruppe, die gebildet wird, aus
- Homopolymeren und nichtionischen Copolymeren des N-Vinylpyrrolidons,
- nichtionischen Copolymeren des Isobutens,
- nichtionischen Copolymeren des Maleinsäureanhydrids.

Geeignete Polyvinylpyrrolidone sind beispielsweise Handelsprodukte wie Luviskol^{®} K 90 oder Luviskol^{®} K 85 der Firma BASF SE.

Geeignetes Polyvinylacetat wird beispielsweise unter dem Handelsnamen Vinac^{®} als Emulsion von der Firma Air Products vertrieben.

Erfindungsgemäße Mittel, die als festigendes nichtionisches Polymer mindestens ein Polymer ausgewählt aus der Gruppe, die gebildet wird aus
- Copolymer aus Maleinsäureanhydrid und Methylvinylether,
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
oder Gemische aus diesen Polymeren enthalten, sind erfindungsgemäß ganz besonders bevorzugt. Dabei sind wiederum solche erfindungsgemäßen Mittel bevorzugt, die als festigendes nichtionisches Polymer mindestens ein Polymer ausgewählt aus der Gruppe, die gebildet wird aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
   oder Gemische aus diesen Polymeren enthalten.

Wenn Copolymere aus N-Vinylpyrrolidon und Vinylestern von Carbonsäuren mit 2 bis 18 Kohlenstoffatomen, insbesondere aus N-Vinylpyrrolidon und Vinylacetat eingesetzt werden, ist es wiederum bevorzugt, wenn das Molverhältnis der aus dem Monomer N-Vinylpyrrolidon enthaltenen Struktureinheiten zu den aus dem Monomer Vinylestern von Carbonsäuren mit 2 bis 18 Kohlenstoffatomen (insbesondere Vinylacetat) enthaltenen Struktureinheiten des Polymers im Bereich von 20 zu 80 bis 80 zu 20, insbesondere von 30 zu 70 bis 60 zu 40, liegt.

Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Die erfindungsgemäßen pulverförmigen kosmetischen Mittel enthalten bevorzugt als filmbildendes Polymer der Komponente (b) mindestens ein kationisches festigendes Polymer. Diese weisen wiederum bevorzugt mindestens eine Lactam-Struktureinheit auf. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein erfindungsgemäß bevorzugt geeignetes kationisches festigendes Polymer ist mindestens ein kationisches festigendes Polymer, das mindestens ein Strukturelement der Formel (M9) und gegebenenfalls zusätzlich mindestens ein Strukturelement der Formel (M10) enthält 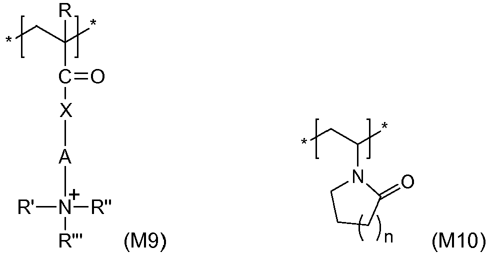 worin
- R: für ein Wasserstoffatom oder eine Methylgruppe steht,
- R', R" und R": unabhängig voneinander stehen für eine (C₁ bis C₃₀)-Alkylgruppe,
- X: steht für ein Sauerstoffatom oder eine Gruppe NH,
- A: steht für eine Ethan-1,2,-diylgruppe oder eine Propan-1,3-diylgruppe,
- n: 1 oder 3 bedeutet.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Mindestens eine der folgenden Verbindungen ist bevorzugt als besagtes kationisches festigendes Polymer einsetzbar
- Homopolymer des 2-(N,N,N-Trimethylammoniumethyl)methacrylats (insbesondere das Chlorid mit der INCI-Bezeichnung Polyquaterium-37, beispielsweise unter dem Handelsnamen Ultragel 300 (BASF SE).
- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN (Firma BASF SE),
- Copolymere aus N-Vinylpyrrolidon, N-(3-Dimethylaminopropyl)methacrylamid und 3-(Methacryloylamino)propyl-lauryl-dimethylammoniumchlorid (INCI-Bezeichnung:
   Polyquaternium-55), welches beispielsweise unter dem Handelsnamen Styleze W 10 oder W 20 (10 bzw. 20 Gew.-% Aktivsubstanz in Ethanol-Wasser-Gemisch) von der Firma ISP vertrieben wird.
- Copolymere aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-Wasser-Gemisch) von der Firma ISP vertrieben wird.

Als im Sinne der Erfindung besonders bevorzugt verwendbare kationische Polymere dienen weiterhin solche kationischen festigenden Copolymere, die mindestens ein Strukturelement der Formel (M11) 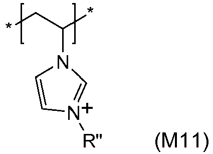
worin R für eine (C₁ bis C₄)-Alkylgruppe, insbesondere eine Methylgruppe, steht,
und zusätzlich mindestens ein weiteres kationisches und/oder nichtionisches Strukturelement aufweisen.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum erfindungsgemäß bevorzugt, wenn als zusätzliches kationisches festigendes Polymer, mindestens ein Copolymer (c1) enthalten ist, das neben mindestens einem Strukturelement der Formel (M11) zusätzlich ein Strukturelement der Formel (M6) umfasst 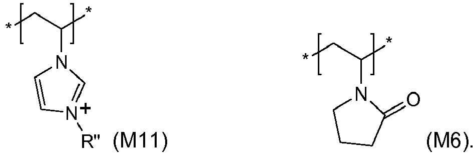
worin R" für eine (C₁ bis C₄)-Alkylgruppe, insbesondere eine Methylgruppe, steht.
Zur Kompensation der positiven Polymerladung der Copolymere (c1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M11) und 70 bis 90 Mol.-%, vorzugsweise 75 bis 85 Mol.-% und insbesondere 80 Mol.-% Struktureinheiten gemäß Formel (M6).

Hierbei ist besonders bevorzugt, wenn die Copolymere (c1) neben Polymereinheiten, die aus dem Einbau der genannten Struktureinheiten gemäß Formel (M11) und (M6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M11) mit R" = Methyl und (M6) aufgebaut.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich.

Zusätzlich zu dem bzw. den Copolymer(en) (c1) oder an dessen bzw. deren Stelle können die erfindungsgemäßen kosmetischen Mittel auch Copolymere (c2) enthalten, die ausgehend vom Copolymer (c1) als zusätzliche Struktureinheiten Struktureinheiten der Formel (M7) aufweisen

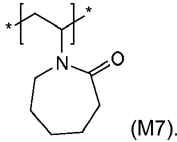

Weitere besonders bevorzugte erfindungsgemäße kosmetische Mittel sind somit dadurch gekennzeichnet, daß sie als kationisches festigendes Polymer mindestens ein Copolymer (c2) enthalten, das mindestens eine Struktureinheit gemäß Formel (M11-a) und mindestens eine Struktureinheit gemäß Formel (M6) und mindestens eine Struktureinheit gemäß Formel (M7) enthält

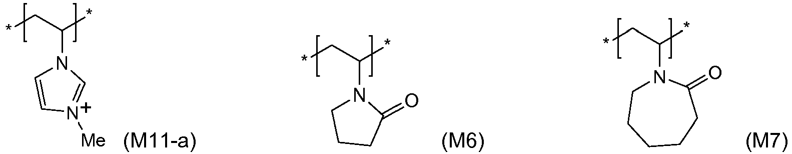

Auch hierbei ist besonders bevorzugt, wenn die Copolymere (c2) neben Polymereinheiten, die aus dem Einbau der genannten Struktureinheiten gemäß Formel (M11-a), (M6) und (M7) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M11-a), (M6) und (M7) aufgebaut.

Zur Kompensation der positiven Polymerladung der Komponente (c2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M11-a) und 30 bis 50 Mol.-%, vorzugsweise 35 bis 45 Mol.-% und insbesondere 40 Mol.-% Struktureinheiten gemäß Formel (M6) und 40 bis 60 Mol.-%, vorzugsweise 45 bis 55 Mol.-% und insbesondere 60 Mol.-% Struktureinheiten gemäß Formel (M7).

Zusätzlich zu dem bzw. den Copolymer(en) (c1) und/oder (c2) oder an dessen bzw. deren Stelle können die erfindungsgemäßen kosmetischen Mittel als festigendes kationisches Polymer auch Copolymere (c3) enthalten, die als Struktureinheiten Struktureinheiten der Formeln (M11-a) und (M6) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße kosmetische Mittel sind dadurch gekennzeichnet, daß sie als zusätzliches kationisches festigendes Polymer mindestens ein Copolymer (c3) enthalten, das mindestens eine Struktureinheit gemäß Formel (M11-a) und mindestens eine Struktureinheit gemäß Formel (M6) und mindestens eine Struktureinheit gemäß Formel (M10) und mindestens eine Struktureinheit gemäß Formel (M12) enthält

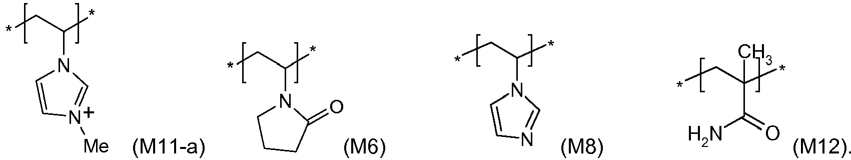

Auch hierbei ist besonders bevorzugt, wenn die Copolymere (c3) neben Polymereinheiten, die aus dem Einbau der genannten Struktureinheiten gemäß Formel (M11-a), (M6), (M8) und (M12) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formel (M11-a), (M6), (M8) und (M12) aufgebaut.

Zur Kompensation der positiven Polymerladung der Komponente (c3) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (M11-a) und 45 bis 65 Mol.-%, vorzugsweise 50 bis 60 Mol.-% und insbesondere 55 Mol.-% Struktureinheiten gemäß Formel (M6) und 1 bis 20 Mol.-%, vorzugsweise 5 bis 15 Mol.-% und insbesondere 10 Mol.-% Struktureinheiten gemäß Formel (M8) und 20 bis 40 Mol.-%, vorzugsweise 25 bis 35 Mol.-% und insbesondere 29 Mol.-% Struktureinheiten gemäß Formel (M12).

Weitere besonders bevorzugte erfindungsgemäße kosmetische Mittel sind dadurch gekennzeichnet, daß sie als kationisches festigendes Polymer mindestens ein Copolymer (c4) enthalten, umfassend mindestens eine Struktureinheit der Formel (M6), mindestens eine Struktureinheit der Formel (M13), mindestens eine Struktureinheit der Formel (M8) und mindestens eine Struktureinheit der Formel (M11), 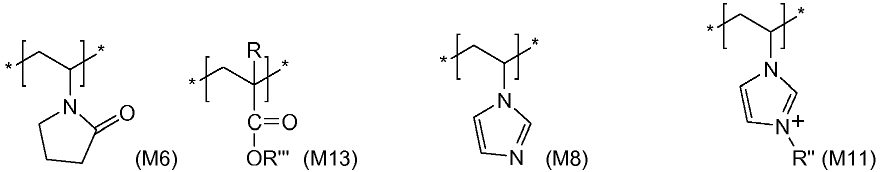 worin
R" für eine (C₁ bis C₄)-Alkylgruppe, insbesondere eine Methylgruppe, steht,
R für ein Wasserstoffatom oder eine Methylgruppe steht,
R"' für ein Wasserstoffatom oder ein Äquivalent eines physiologisch verträglichen Kations steht. Jedes physiologisch verträgliches Anion (wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat) kann zur Kompensation der positiven Ladung der Struktureinheit (IV) dienen.

Beispiele für erfindungsgemäße (C₁ bis C₄)-Alkylgruppen sind Methyl, Ethyl, Propyl, Isopropyl, Butyl, Sec-Butyl, Isobutyl, tert-Butyl.

Es ist erfindungsgemäß bevorzugt, wenn das besagte Copolymer bei einem pH-Wert von pH 7 eine Ladungsdichte der kationischen Ladung von 0.8 meq/g aufweist.

Es ist erfindungsgemäß bevorzugt, wenn R gemäß Formel (M13) für eine Methylgruppe steht. Es ist erfindungsgemäß bevorzugt, wenn R"' gemäß Formel (M13) für ein Wasserstoffatom steht. Es ist erfindungsgemäß bevorzugt, wenn R" gemäß Formel (M11) für eine Methylgruppe steht.

Ein erfindungsgemäß besonders bevorzugtes Copolymer umfasst mindestens eine Struktureinheit der Formel (M6), mindestens eine Struktureinheit der Formel (M13-a), mindestens eine Struktureinheit der Formel (M8) und mindestens eine Struktureinheit der Formel (M11-a),

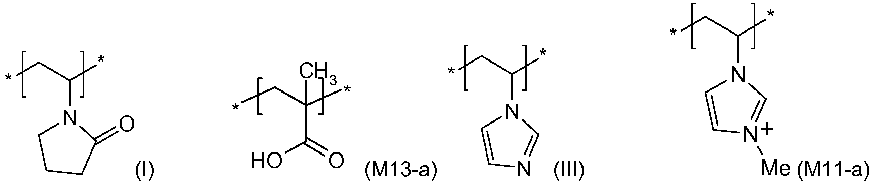

Ganz besonders bevorzugt enthalten die erfindungsgemäßen Mittel ein Copolymer aus N-Vinylpyrrolidon, N-Vinylimidazol, N-Methyl-N'-vinylimidazoliumchlorid und Methacrylsäure als besagtes Copolymer.
Es ist erfindungsgemäß am bevorzugtesten, wenn als Copolymer das mit der INCI-Bezeichnung Polyquaternium-86 bezeichnete Copolymer in den erfindungsgemäßen Mitteln enthalten ist. INCI steht als Abkürzung für International Nomenclature of Cosmetic Ingredients. Solche Copolymere werden beispielsweise von der Firma BASF SE unter dem Handelsnamen Luvigel^{®} Advanced in Form eines weißen Pulvers vertrieben.

Unter den festigenden Polymeren ausgewählt aus den kationischen Polymeren mit mindestens einem Strukturelement der obigen Formel (M11-a), gelten als bevorzugt:
- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat^{®} FC 370, Luviquat^{®} FC 550, Luviquat^{®} FC 905 und Luviquat^{®} HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat^{®} Supreme (BASF SE)),
- Copolymer aus N-Vinylpyrrolidon, N-Vinylimidazol, N-Methyl-N'-vinylimidazoliumchlorid und Methacrylsäure (wie beispielsweise das mit der INCI-Bezeichnung Polyquaternium-86 unter der Handelsbezeichnung Luvigel^{®} Advanced (BASF SE)).
   sowie Gemische aus diesen Polymeren.
Weitere in den erfindungsgemäßen kosmetischen Mitteln bevorzugt einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Ganz besonders bevorzugte pulverförmige kosmetische Mittel enthalten als festigendes Polymer der Komponente (b) mindestens ein festigendes Polymer, welches mindestens eine Struktureinheit der Formel (III) und mindestens eine Struktureinheit der Formel (IV) umfasst, 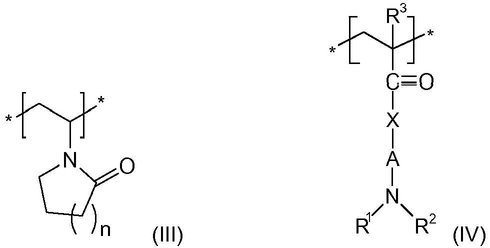
n steht für 1, 2 oder 3,
R¹ und R² stehen unabhängig voneinander für eine (C₁ bis C₆)-Alkylgruppe (insbesondere für Methyl)
A steht für Ethan-1,2-diyl oder Propan-1,3-diyl,
X steht für ein Sauerstoffatom oder eine Gruppe NH,
R³ steht für ein Wasserstoffatom oder Methyl.

Als im Sinne der Erfindung bevorzugt geeignete festigende Polymere der Komponente (b) gelten solche, die als Struktureinheit der Formel (IV) mindestens eine Struktureinheit der Formeln (M1-1) bis (M1-8) aufweisen

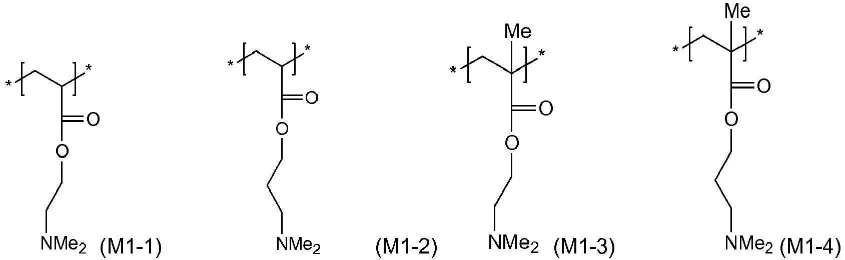

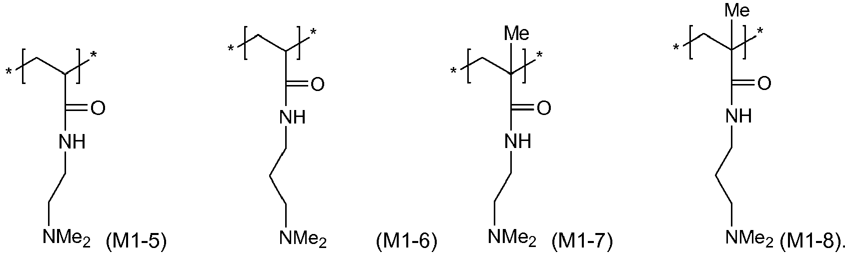

Dabei gilt wiederum die Gruppe der Polymere
- Vinylcaprolactam/Vinylpyrrolidon/Dimethylaminoethylmethacrylat-Copolymer (beispielsweise INCI-Bezeichnung: Vinyl Caprolactam/PVP/Di-methylaminoethyl Methacrylate Copolymer unter dem Handelsnamen Gaffix^{®} VC 713 (ISP)),
- Vinylpyrrolidon/Vinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex^{®} SF-40 (ISP)),
- Vinylcaprolactam/Vinylpyrrolidon/Dimethylaminoethylmethacrylat-Copolymer (beispielsweise als Pulver in Form des Handelsprodukts Advantage S mit der INCI-Bezeichnung: Vinyl Caprolactam/VP/Dimethylaminoethyl Methacrylate Copolymer (ISP)),
- Vinylpyrrolidon/Dimethylaminopropylmethacrylamid-Copolymer (beispielsweise INCI-Bezeichnung: VP/DMAPA Acrylates Copolymer unter dem Handelsnamen Styleze CC-10 (ISP)),
   als bevorzugte Liste zur Auswahl mindestens eines oder mehrerer festigender Polymere daraus.

Die kationischen Polymere sind erfindungsgemäß bevorzugt in Mengen von 2,0 Gew.-% bis 95 Gew.-%, insbesondere von 5,0 Gew.-% bis 95,0 Gew.-%, ganz besonders bevorzugt von 7,5 bis 95,0 Gew.-%, jeweils bezogen auf das Gesamtgewicht der erfindungsgemäßen kosmetischen Mittel, enthalten.

Die erfindungsgemäßen kosmetischen Mittel können als festigendes Polymer auch mindestens ein amphoteres festigendes Polymer enthalten. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻Gruppen enthalten, und solche Polymere zusammengefaßt, die -COOH- oder SO₃H-Gruppen und quartäre Ammoniumgruppen enthalten.
Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Alkylestern darstellt.

Weiterhin können als festigende Polymere der Komponente (b) bevorzugt mindestens ein anionisches festigendes Polymer eingesetzt werden. Bei den anionischen Polymeren handelt es sich um anionische Polymere, welche bevorzugt Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen.

Innerhalb dieser Ausführungsform ist es bevorzugt, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Weitere bevorzugt einsetzbare anionische Polymere werden ausgewählt aus der Gruppe, die gebildet wird, aus
- Copolymeren aus Vinylacetat und Crotonsäure (wie sie beispielsweise als Handelsprodukt Aristoflex^{®} A 60 mit der INCI-Bezeichnung VA/Crotonates Copolymer von der Firma CIBA in einer 60 Gew.-%-igen Dispersion in Isopropanol-Wasser vermarktet werden),
- Copolymeren aus Ethylacrylat und Methacrylsäure (wie sie beispielsweise unter dem Handelsnamen Luviflex^{®} Soft mit einer Säurezahl von 84 bis 105 unter der INCI-Bezeichnung Acrylates Copolymer in einer ca. 20 bis 30 Gew.-%igen Dispersion in Wasser von der Firma BASF SE vertrieben werden),
- Polyurethanen mit mindestens einer Carboxylgruppe (wie beispielsweise ein Copolymer aus Isophthalsäure, Adipinsäure, 1,6-Hexandiol, Neopentylglykol und Isophorondiisocyanat wie es unter dem Handelsnamen Luviset PUR mit der INCI-Bezeichnung Polyurethane-1 von der Firma BASF SE vertrieben wird).

Ferner ist es bevorzugt, wenn die erfindungsgemäßen kosmetischen Mittel bezogen auf ihr Gewicht nicht mehr als 8 Gew.-%, insbesondere nicht mehr als 5 Gew.-%, einer Flüssigkeit enthalten.

Die erfindungsgemäßen kosmetischen Mittel liegen in Pulverform vor. Pulverförmig im Sinne der Erfindung sind kosmetische Mittel, deren Partikel unter eigenem Gewicht frei rieselfähig sind (vgl. DIN EN ISO 6186: 1998-08). Partikel sind im Sinne der Erfindung als Korn vorliegende Teilchen (vgl. DIN 66160: 1992-09) von Festkörpern.

Es sei explizit darauf hingewiesen, dass die zuvor als bevorzugt genannten Vertreter der Komponente (a) und der Komponente (b) des erfindungsgemäßen Mittels (*vide supra*) für die erfindungsgemässe Ausführungsform als Pulver gelten.

Im Rahmen der erfindungsgemäßen Pulver hat es sich wiederum als besonders vorteilhaft erwiesen, wenn der volumenbezogene mittlere Partikeldurchmesser (d0,5 Volumenbezogen, mittels Laserbeugung bestimmt) des Pulvers in einem Bereich von 10 µm bis 800 µm, insbesondere von 25 µm bis 500 µm, liegt.

Unter keratinhaltigen Fasern sind dabei erfindungsgemäß Pelze, Wolle, Federn und insbesondere menschliche Haare zu verstehen.

Die erfindungsgemäßen Mittel in Pulverform können sehr einfach dosiert werden. Sie lassen sich sehr gleichmäßig in Gegenwart einer geringfügigen Wassermenge d.h. durch vorheriges auftragen auf eine feuchte Oberfläche einer Applikationshilfe und/oder durch Anwendung auf feuchtem Haar, auf den keratinhaltigen Fasern verteilen. Die geringe Menge an Lösemittel auf dem feuchten Haar oder der feuchten Oberfläche einer Applikationshilfe reicht für die gezielte Benetzung der Haarfasern mit dem Kosmetikum aus. Es findet keine Klumpenbildung statt.

Die erfindungsgemäßen Pulver können zusätzlich mindestens ein Wachs enthalten. Erfindungsgemäße Wachse weisen bei 1013 mbar einen Schmelzpunkt in einem Bereich von 38°C bis 95°C auf.

Bevorzugt werden die Wachse aus pflanzlichen, tierischen und mineralischen Wachsen ausgewählt, wobei solche Wachse bevorzugt sind, die einen Schmelzpunkt im Bereich von 50°C bis 90°C, aufweisen.

Erfindungsgemäß besonders bevorzugt, enthält das erfindungsgemäße kosmetische Mittel mindestens ein Wachs, ausgewählt aus mindestens einem Wachs der Gruppe Bienenwachs (Cera Alba), Carnaubawachs, Candelillawachs, Wachs aus Reiskleie (Rice Bran Wax), Montanwachs, Paraffinwachs, mikrokristallinem Paraffinwachs, Ozokeritwachs und Cetylpalmitat.

Die erfindungsgemäße Lehre umfasst auch den kombinierten Einsatz von mehreren Wachsen. So kann ein Zusatz von Carnaubawachs dazu verwendet werden, um Schmelz- und Tropfpunkt eines anderen Wachses zu erhöhen und dessen Klebrigkeit zu vermindern. Entsprechend sind eine Reihe von Wachsmischungen, gegebenenfalls in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Kahlwax 6240 (Hydrogenated Vegetable Oil) und "Weichceresin^{®} FL 400" (ein Vaseline/Vaselinöl/Wachs-Gemisch mit einem Schmelzpunkt von 50-54 °C; Hersteller: Parafluid Mineralölgesellschaft) sind Beispiele für erfindungsgemäß bevorzugt eingesetzte Mischungen.

Es hat sich als besonders bevorzugt erwiesen, wenn wachshaltige erfindungsgemäße Mittel gemeinsam mit mindestens einem Wachs zusätzlich mindestens einen Emulgator enthalten. Im Rahmen dieser Ausführungsform können auch flüssige Emulgatoren eingesetzt werden, solange diese in Abmischung mit dem mindestens einem Wachs z.B. durch Sorption eine pulverförmige Verbindung ergeben.

Bevorzugte Emulgatoren sind nichtionisch und enthalten als hydrophile Gruppe z.B. eine Polyolgruppe, eine Polyalkylenglykolethergruppe oder eine Kombination aus Polyol- und Polyglykolethergruppe. Das erfindungsgemäße kosmetische Mittel enthält bevorzugt neben mindestens einem Wachs zusätzlich mindestens einen nichtionischen Emulgator ausgewählt aus mindestens einer Verbindung aus der Gruppe, die gebildet wird aus
- Anlagerungsprodukten von 2 bis 100 Mol Ethylenoxid und/oder 1 bis 5 Mol Propylenoxid an gesättigte oder ungesättigte, lineare oder verzweigte Fettalkohole mit 8 bis 30 C-Atomen, an Fettsäuren mit 8 bis 30 C-Atomen,
- mit einem Methyl- oder C₂ - C₆ - Alkylrest endgruppenverschlossenen Anlagerungsprodukten von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diestern von Anlagerungsprodukten von 1 bis 30 Mol Ethylenoxid an Glycerin,
- Anlagerungsprodukten von 5 bis 60 Mol Ethylenoxid an Rizinusöl und gehärtetes Rizinusöl,
- Polyolfettsäureestern, wie beispielsweise das Handelsprodukt Hydagen^{®} HSP (Cognis) oder Sovermol - Typen (Cognis),
- alkoxilierten Triglyceriden,
- alkoxilierten Fettsäurealkylestern der Formel (E4-I)

   R¹CO-(OCH₂CHR₂)_{w}OR₃ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxiden,
- Hydroxymischethern,
- Sorbitanfettsäureestern und Anlagerungeprodukten von Ethylenoxid an Sorbitanfettsäureester wie beispielsweise die Polysorbate,
- Zuckerfettsäureestern und Anlagerungsprodukten von Ethylenoxid an Zuckerfettsäureester,
- Anlagerungsprodukten von Ethylenoxid an Fettsäurealkanolamiden und Fettaminen,
- Zuckertensiden vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.

Die erfindungsgemässen Pulver können zusätzlich pulverförmige Metalloxide enthalten. Diese Metalloxide können hydrophil oder hydrophob sein.

Bevorzugt geeignete Metalloxide werden erfindungsgemäß ausgewählt aus mindestens einem Vertreter der Gruppe, die gebildet wird aus Silikaten, Aluminiumsilikaten, Titandioxid, Zinkoxid sowie Siliziumdioxid.

Besonders bevorzugte Aluminiumsilikate (auch Alumosilikate genannt) werden ausgewählt unter Phyllosilikaten, Tectosilikaten oder Gemischen daraus.

Bevorzugt geeignete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonit, Phlogopit, Biotit, Lepidolith, Margarit, Smektit (hier insbesondere unter Montmorrilionit, Saponit, Nontronit, Hectorit)).

Bevorzugt geeignete Tectosilikate werden ausgewählt unter Feldspatmineralien (insbesondere Albit, Orthoklas, Anorthit, Leucit, Sodalith, Hauyn, Labradorit, Lasurit, Nosean, Nephelin), Zeolithen.

Die erfindungsgemässen Pulver können zusätzlich pulverförmige Salze enthalten. Hierbei eignen sich Phosphate, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- bzw. Pentakaliumtriphosphat (Natrium- bzw. Kaliumtripolyphosphat) eine gute Eignung.

Alkalimetallphosphate ist dabei die summarische Bezeichnung für die Alkalimetall- (insbesondere Natrium- und Kalium-) Salze der verschiedenen Phosphorsäuren, bei denen man Metaphosphorsäuren (HPO₃)ₙ und Orthophosphorsäure H₃PO₄ neben höhermolekularen Vertretern unterscheidet. Als Beispiele für solche Phosphate sind das Pentanatriumtriphosphat, Na₅P₃O₁₀ (Natriumtripolyphosphat) sowie das entsprechende Kaliumsalz Pentakaliumtriphosphat, K₅P₃O₁₀ (Kaliumtripolyphosphat) zu nennen. Erfindungsgemäß bevorzugt eingesetzt werden weiterhin die Natriumkaliumtripolyphosphate.

Als gleichfalls geeignet haben sich Mineralsalze einwertiger Metallionen, wie beispielsweise Na₂SO₄ oder Natriumchlorid erwiesen.

Die erfindungsgemässen Pulver können zusätzlich Dextrine enthalten, wie beispielsweise Oligomere bzw. Polymere von Kohlenhydraten,
die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren.

Die Ausführungsformen (A) bis (H) sind im Sinne der Erfindung ganz besonders bevorzugt:
(A): Pulverförmiges kosmetisches Mittel, enthaltend als Komponente (a) mindestens ein Polymer, umfassend mindestens eine Struktureinheit der Formel (I) und mindestens eine Struktureinheit der Formel (II), 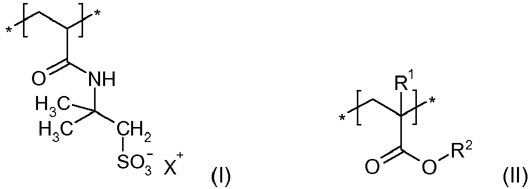
   worin X⁺ für ein physiologisch verträgliches Kation (insbesondere Na⁺) steht,
   R¹ für ein Wasserstoffatom oder eine Methylgruppe steht und
   R² für eine (C₂ bis C₆)-Hydroxyalkylgruppe steht,
      und als Komponente (b) mindestens ein festigendes Polymer umfassend mindestens eine Lactam-Struktureinheit.
(B): Pulverförmiges kosmetisches Mittel, enthaltend als Komponente (a) mindestens ein Polymer, umfassend mindestens eine Struktureinheit der Formel (I) und mindestens eine Struktureinheit der Formel (II), 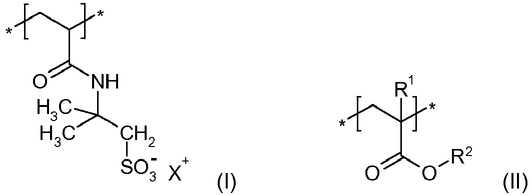
   worin X⁺ für ein physiologisch verträgliches Kation (insbesondere Na⁺) steht,
   R¹ für ein Wasserstoffatom oder eine Methylgruppe steht und
   R² für eine (C₂ bis C₆)-Hydroxyalkylgruppe steht,
      und als Komponente (b) mindestens ein festigendes Polymer umfassend mindestens eine Struktureinheit der Formel (III) und mindestens eine Struktureinheit der Formel (IV), 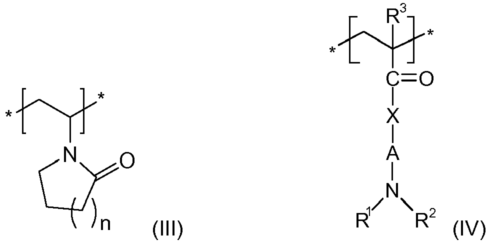
      n steht für 1, 2 oder 3,
      R¹ und R² stehen unabhängig voneinander für eine (C₁ bis C₆)-Alkylgruppe (insbesondere für Methyl)
      A steht für Ethan-1,2-diyl oder Propan-1,3-diyl,
      X steht für ein Sauerstoffatom oder eine Gruppe NH,
      R³ steht für ein Wasserstoffatom oder Methyl.
(C): Pulverförmiges kosmetisches Mittel, enthaltend als Komponente (a) mindestens ein Polymer, umfassend mindestens eine Struktureinheit der Formel (I) und mindestens eine Struktureinheit der Formel (II), 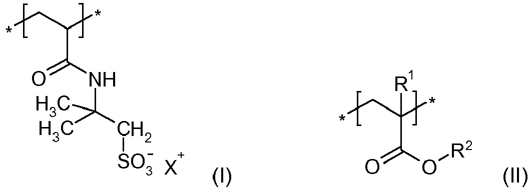
   worin X⁺ für ein physiologisch verträgliches Kation (insbesondere Na⁺) steht,
   R¹ für ein Wasserstoffatom oder eine Methylgruppe steht und
   R² für 2-Hydroxyethyl steht,
      und als Komponente (b) mindestens ein festigendes Polymer umfassend mindestens eine Lactam-Struktureinheit.
(D): Pulverförmiges kosmetisches Mittel, enthaltend als Komponente (a) mindestens ein Polymer, umfassend mindestens eine Struktureinheit der Formel (I) und mindestens eine Struktureinheit der Formel (II), 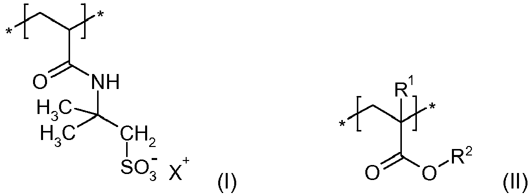
   worin X⁺ für ein physiologisch verträgliches Kation (insbesondere Na⁺) steht,
   R¹ für ein Wasserstoffatom oder eine Methylgruppe steht und
   R² für 2-Hydroxyethyl steht,
      und als Komponente (b) mindestens ein festigendes Polymer umfassend mindestens eine Struktureinheit der Formel (III) und mindestens eine Struktureinheit der Formel (IV), 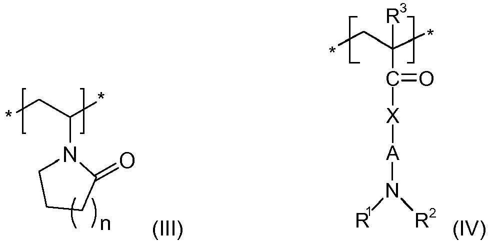
      n steht für 1, 2 oder 3,
      R¹ und R² stehen unabhängig voneinander für eine (C₁ bis C₆)-Alkylgruppe (insbesondere für Methyl)
      A steht für Ethan-1,2-diyl oder Propan-1,3-diyl,
      X steht für ein Sauerstoffatom oder eine Gruppe NH,
      R³ steht für ein Wasserstoffatom oder Methyl.
(E): Pulverförmiges kosmetisches Mittel, enthaltend bezogen auf das Gesamtgewicht des kosmetischen Mittels als Komponente (a) von 2,0 Gew.-% bis 95 Gew.-%, insbesondere von 5,0 Gew.-% bis 95,0 Gew.-%, besonders bevorzugt von 7,5 bis 95,0 Gew.-%, mindestens eines Polymers, umfassend mindestens eine Struktureinheit der Formel (I) und mindestens eine Struktureinheit der Formel (II), 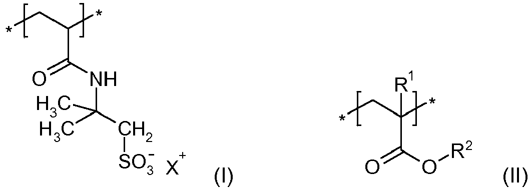
   worin X⁺ für ein physiologisch verträgliches Kation (insbesondere Na⁺) steht, R¹ für ein Wasserstoffatom oder eine Methylgruppe steht und
   R² für eine (C₂ bis C₆)-Hydroxyalkylgruppe steht,
      und als Komponente (b) 5 und 95 Gew.-%, insbesondere von 0,1 bis 50 Gew.-%, besonders bevorzugt von 5,0 bis 40,0 Gew.-% und ganz besonders bevorzugt von 10,0 bis 30,0 Gew.-% mindestens eines festigenden Polymers, umfassend mindestens eine Lactam-Struktureinheit.
(F): Pulverförmiges kosmetisches Mittel, enthaltend bezogen auf das Gesamtgewicht des kosmetischen Mittels als Komponente (a) von 2,0 Gew.-% bis 95 Gew.-%, insbesondere von 5,0 Gew.-% bis 95,0 Gew.-%, besonders bevorzugt von 7,5 bis 95,0 Gew.-%, mindestens eines Polymers, umfassend mindestens eine Struktureinheit der Formel (I) und mindestens eine Struktureinheit der Formel (II), 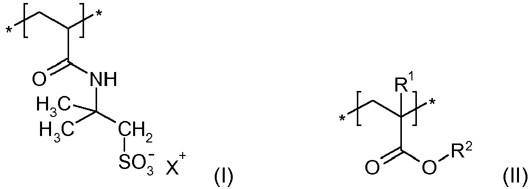
   worin X⁺ für ein physiologisch verträgliches Kation (insbesondere Na⁺) steht,
   R¹ für ein Wasserstoffatom oder eine Methylgruppe steht und
   R² für eine (C₂ bis C₆)-Hydroxyalkylgruppe steht,
      und als Komponente (b) 5 und 95 Gew.-%, insbesondere von 0,1 bis 50 Gew.-%, besonders bevorzugt von 5,0 bis 40,0 Gew.-% und ganz besonders bevorzugt von 10,0 bis 30,0 Gew.-% mindestens eines festigenden Polymers, umfassend mindestens eine Struktureinheit der Formel (III) und mindestens eine Struktureinheit der Formel (IV), 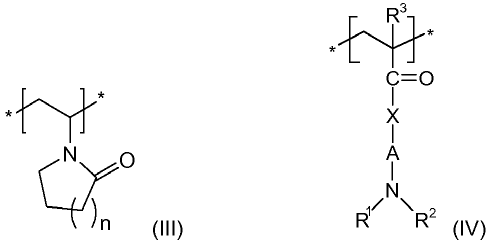
      n steht für 1, 2 oder 3,
      R¹ und R² stehen unabhängig voneinander für eine (C₁ bis C₆)-Alkylgruppe (insbesondere für Methyl)
      A steht für Ethan-1,2-diyl oder Propan-1,3-diyl,
      X steht für ein Sauerstoffatom oder eine Gruppe NH,
      R³ steht für ein Wasserstoffatom oder Methyl.
(G): Pulverförmiges kosmetisches Mittel, enthaltend bezogen auf das Gesamtgewicht des kosmetischen Mittels als Komponente (a) von 2,0 Gew.-% bis 95 Gew.-%, insbesondere von 5,0 Gew.-% bis 95,0 Gew.-%, besonders bevorzugt von 7,5 bis 95,0 Gew.-%, mindestens eines Polymers, umfassend mindestens eine Struktureinheit der Formel (I) und mindestens eine Struktureinheit der Formel (II), 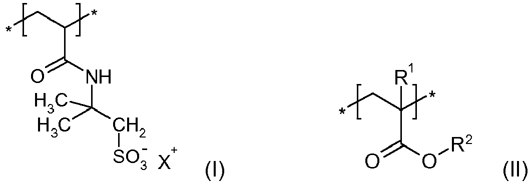
   worin X⁺ für ein physiologisch verträgliches Kation (insbesondere Na⁺) steht,
   R¹ für ein Wasserstoffatom oder eine Methylgruppe steht und
   R² für 2-Hydroxyethyl steht,
      und als Komponente (b) 5 und 95 Gew.-%, insbesondere von 0,1 bis 50 Gew.-%, besonders bevorzugt von 5,0 bis 40,0 Gew.-% und ganz besonders bevorzugt von 10,0 bis 30,0 Gew.-% mindestens eines festigenden Polymers, umfassend mindestens eine Lactam-Struktureinheit.
(H): Pulverförmiges kosmetisches Mittel, bezogen auf das Gesamtgewicht des kosmetischen Mittels als Komponente (a) von 2,0 Gew.-% bis 95 Gew.-%, insbesondere von 5,0 Gew.-% bis 95,0 Gew.-%, besonders bevorzugt von 7,5 bis 95,0 Gew.-%, mindestens eines Polymers, umfassend mindestens eine Struktureinheit der Formel (I) und mindestens eine Struktureinheit der Formel (II), 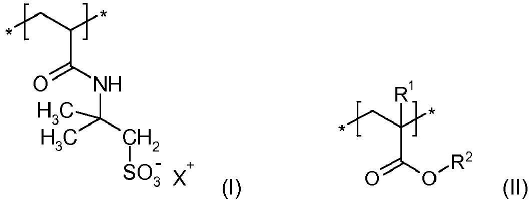
   worin X⁺ für ein physiologisch verträgliches Kation (insbesondere Na⁺) steht,
   R¹ für ein Wasserstoffatom oder eine Methylgruppe steht und
   R² für 2-Hydroxyethyl steht,
      und als Komponente (b) 5 und 95 Gew.-%, insbesondere von 0,1 bis 50 Gew.-%, besonders bevorzugt von 5,0 bis 40,0 Gew.-% und ganz besonders bevorzugt von 10,0 bis 30,0 Gew.-% mindestens eines festigenden Polymers, umfassend mindestens eine Struktureinheit der Formel (III) und mindestens eine Struktureinheit der Formel (IV), 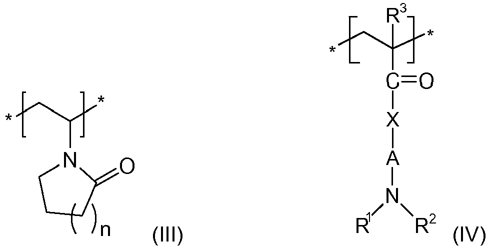
      n steht für 1, 2 oder 3,
      R¹ und R² stehen unabhängig voneinander für eine (C₁ bis C₆)-Alkylgruppe (insbesondere für Methyl)
      A steht für Ethan-1,2-diyl oder Propan-1,3-diyl,
      X steht für ein Sauerstoffatom oder eine Gruppe NH,
      R³ steht für ein Wasserstoffatom oder Methyl.

Für die Ausführungsformen (A) bis (H) gelten die zuvor als bevorzugt gekennzeichneten Ausführungsformen der darin genannten Merkmale (*vide supra*) *mutatis mutandis* ebenso als bevorzugt. Ferner sind die als bevorzugt genannten mittleren Partikeldurchmesser im Rahmen der Ausführungsformen (A) bis (H) bevorzugt.

Die erfindungsgemäßen pulverförmigen kosmetischen Mittel können in nahezu beliebigen Behältnissen konfektioniert werden. Es muss lediglich sichergestellt werden, dass die mechanische Belastung des Pulvers bei der Entnahme des Mittels nicht so hoch ist, dass bereits bei der Entnahme das Pulver in flüssige Form überführt wird. Geeignet sind beispielsweise Tiegel, Flaschen oder auch Tetrapacks, wobei das Behältnis beispielsweise mit einer Schütt- und Dosiervorrichtung ausgestaltet werden kann.

Ein zweiter Gegenstand der Erfindung ist die Verwendung eines kosmetischen Mittels des ersten Erfindungsgegenstandes zur temporären Verformung keratinhaltiger Fasern, insbesondere menschlicher Haare. Das erfindungsgemäße kosmetische Mittel kann bei der Anwendung in Pulverform vorliegen oder einen wässrigen kosmetischen Träger umfassen. Bevorzugte wässrige kosmetische Träger sind wässrige oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Ein dritter Erfindungsgegenstand ist ein Verfahren zur kosmetischen Behandlung menschlicher Haare, in dem ein kosmetisches Mittel des ersten Erfindungsgegenstandes auf gegebenenfalls feuchtes Haar aufgetragen wird und auf dem Haar belassen wird.

Ein erfindungsgemäß bevorzugtes Verfahren dieser Art ist ein Verfahren zur kosmetischen Behandlung menschlicher Haare, in dem
(i) eine als Applikationshilfe genutzte Oberfläche befeuchtet wird,
(ii) ein pulverförmiges kosmetisches Mittel des ersten Erfindungsgegenstandes auf die befeuchtete Oberfläche der Applikationshilfe aufgetragen wird,
(iii) die Haare mit der Oberfläche aus (ii) zur Applikation des angefeuchteten, besagten kosmetischen Mittels in Kontakt gebracht und zur Frisur gelegt werden,
(iv) die Haare getrocknet werden,
   mit der Maßgabe, dass die Haare nach Schritt (iii) nicht gespült werden.

Als Applikationshilfe zur Applikation der pulverförmigen Zusammensetzung dient beispielsweise die Hand, ein Pinsel, ein Schwamm, ein Tuch, eine Haarbürste, eine Rundbürste, ein Lockenwickler, eine Mascarabürste oder ein Kamm. Dabei ist es erfindungsgemäß bevorzugt, wenn die genutzte Oberfläche der Applikationshilfe die Handinnenfläche ist.

Das in Kontakt bringen gemäß Schritt (iii) ist beispielsweise ein gezieltes Einmassieren des Pulvers in das Haar, kämmen oder bürsten.
Das zur Frisur legen gemäß Schritt (iii) kann in einem Arbeitsgang mit der Applikation der besagten mindestens einen pulverförmigen, haarkosmetisch wirksamen Verbindung erfolgen.

Die Trocknung im Schritt (iv) kann durch trocknen an der Raumluft oder z.B. durch Warmluft eines Föns erfolgen.

Ein weiteres erfindungsgemäß bevorzugtes Verfahren als Ausführungsform des dritten Erfindungsgegenstandes ist ein Verfahren zur kosmetischen Behandlung menschlicher Haare, in dem
(i) die Haare angefeuchtet werden,
(ii) auf die feuchten Haare ein pulverförmiges kosmetisches Mittel des ersten Erfindungsgegenstandes aufgetragen wird,
(iii) die Haare zur Frisur gelegt werden,
(iv) die Haare getrocknet werden,
   mit der Maßgabe, dass die Haare nach Schritt (ii) nicht gespült werden.

Das Anfeuchten des Haars im Schritt (i) kann durch Besprühen der Fasern mit einer Flüssigkeit, bevorzugt mit Wasser, geschehen. Bevorzugterweise werden die Fasern in Schritt (i) mit einem herkömmlichen Shampoo shampooniert, gespült und dann mit einem Handtuch frottiert. Nach Abschluss des Frottierschritts ist das Haar nicht tropfnass, aber es bleibt eine fühlbare Restfeuchtigkeit im Haar zurück.

Die pulverförmige Zusammensetzung wird gemäß Schritt (ii) beispielsweise mittels eines salzstreuerartigen Dosiersystems auf die Haare dosiert aufgetragen.

Das zur Frisur legen gemäß Schritt (iii) kann in einem Arbeitsgang mit der Applikation der besagten mindestens einen pulverförmigen, haarkosmetisch wirksamen Verbindung erfolgen.

Die Trocknung im Schritt (iv) kann durch trocknen an der Raumluft oder z.B. durch Warmluft eines Föns erfolgen.

Die bevorzugten Ausführungsformen des ersten Erfindungsgegenstandes gelten *mutatis mutandis* auch für die Erfindungsgegenstände drei und vier.

### Beispiele

### 1. Herstellung pulverförmiger Stylingmittel

Es wurden wie nachfolgend beschrieben die erfindungsgemäßen, pulverförmigen Stylingmittel E1 bis E10 hergestellt, wobei diese folgende Zusammensetzung aufwiesen:

| **Rohstoff** | **E1** | **E2** | **E3** | **E4** | **E5** |
|---|---|---|---|---|---|
| Sepinov^{®} EMT 10, Pulver | 90,0 | 15,0 | 20,0 | 15,0 | 17,5 |
| Advantage^{®} S, Pulver | 10,0 | 85,0 | 80,0 | 60,0 | 81,9 |
| Parfum | - | - | - | - | 0,6 |

| **Rohstoff** | **E6** | **E7** | **E8** | **E9** | **E10** |
|---|---|---|---|---|---|
| Sepinov^{®} EMT 10, Pulver | 17,5 | 20,0 | 22,5 | 20,0 | 20,0 |
| Advantage^{®} S, Pulver | 82,0 | 79,5 | 77,0 | 78,8 | 78,8 |
| Parfum | 0,5 | 0,5 | 0,5 | 1,2 | 1,2 |

Alle pulverförmigen Rohstoffe wurden in einem Mahlwerk einzeln gemahlen. Dann wurden die Rohstoffe entsprechend gemischt. Das so erhaltene fertige Stylingpulver wurde in Polyethylenflaschen abgefüllt.

### 2.0 Anwendung

### 2.1 auf feuchtes Haar

Es wurden 10 Haarsträhnen mit je einem der oben genannten Stylingmittel behandelt. Dazu wurde die Haarsträhne angefeuchtet und auf die feuchte Strähne das erfindungsgemäße Mittel aufgetragen und einmassiert. Die behandelte Haarsträhne wurde auf einem Brett gestreckt fixiert und trocknen gelassen.

Mit den erfindungsgemäßen Zusammensetzungen E1 bis E10 bekamen die Strähnen einen hervorragenden Halt der Form. Das Haar besaß deutlich mehr Struktur und Textur. Trotz der eingesetzten partikelförmigen Rohstoffe war keine sichtbare Mattierung des Haars zu beobachten. Das Haar behielt seinen natürlichen Glanz.

### 2.2 auf trockenes Haar

Es wurden 10 Haarsträhnen mit der Hand mit je einem der oben genannten Stylingmittel behandelt. Dazu wurde die Handinnenfläche mit Wasser befeuchtet und das erfindungsgemäße Mittel auf die feuchte Handinnenfläche unter Bildung eines Gels verrieben. Das resultierende Gel wurde auf die Haarsträhne aufgetragen und einmassiert. Die behandelte Haarsträhne wurde auf einem Brett gestreckt fixiert und trocknen gelassen.

Das Haar besaß deutlich mehr Struktur und Textur. Trotz der eingesetzten partikelförmigen Rohstoffe war keine sichtbare Mattierung des Haars zu beobachten. Das Haar behielt seinen natürlichen Glanz.

### 3. Verzeichnis der eingesetzten Rohstoffe

Die im Rahmen der Beispiele eingesetzten Rohstoffe sind wie folgt definiert:

| | |
|---|---|
| Sepinov EMT 10, Pulver | Copolymer von 2-Hydroxyethylacrylat und 2-Methyl-2-(1-oxo-2-propenyl)amino)-1-propansulfonsäure Mononatriumsalz (INCI-Bezeichnung: Hydroxyethyl acrylate / Sodium acryloyl dimethyl Taurate Copolymer) (Seppic) |
| Advantage S, Pulver | Vinylcaprolactam/Vinylpyrrolidon/Dimethylaminoethylmethacrylat-Copolymer (INCI-Bezeichnung: Vinyl Caprolactam / VP / Dimethylaminoethyl Methacrylate Copolymer) (ISP) |

## Patentansprüche

1. Pulverförmiges kosmetisches Mittel, enthaltend als Komponente (a) mindestens ein Polymer, umfassend mindestens eine Struktureinheit der Formel (I) und mindestens eine Struktureinheit der Formel (II), 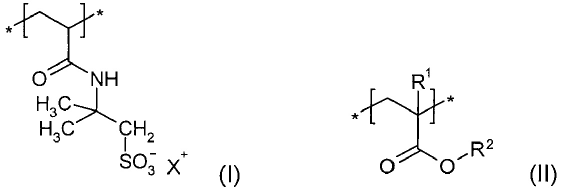
worin X⁺ für ein physiologisch verträgliches Kation (insbesondere Na⁺) steht,
R¹ für ein Wasserstoffatom oder eine Methylgruppe steht und
R² für eine (C₂ bis C₆)-Hydroxyalkylgruppe steht,
und als Komponente (b) mindestens ein festigendes Polymer.

2. Pulverförmiges kosmetisches Mittel nach Anspruch 1, **dadurch gekennzeichnet, dass** R² für eine 2-Hydroxyethylgruppe steht.

3. Pulverförmiges kosmetisches Mittel nach einem der vorstehenden Ansprüche, **dadurch gekennzeichnet, dass** das Polymer der Komponente (a) bezogen auf das Gesamtgewicht der Zusammensetzung in einer Menge von 5 und 95 Gew.-%, besonders bevorzugt von 5,0 bis 40,0 Gew.-% und ganz besonders bevorzugt von 10,0 bis 30,0 Gew.-%, enthalten ist.

4. Pulverförmiges kosmetisches Mittel nach einem der vorstehenden Ansprüche, **dadurch gekennzeichnet, dass** das festigende Polymer der Komponente (b) ausgewählt wird aus Polymeren umfassend mindestens eine Lactam-Struktureinheit.

5. Pulverförmiges kosmetisches Mittel nach einem der vorstehenden Ansprüche, **dadurch gekennzeichnet, dass** das festigende Polymer der Komponente (b) mindestens eine Struktureinheit der Formel (III) und mindestens eine Struktureinheit der Formel (IV) umfasst, 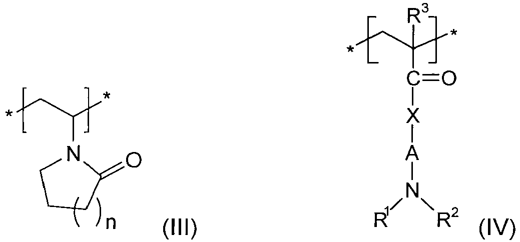
n steht für 1, 2 oder 3,
R¹ und R² stehen unabhängig voneinander für eine (C₁ bis C₆)-Alkylgruppe (insbesondere für Methyl)
A steht für Ethan-1,2-diyl oder Propan-1,3-diyl,
X steht für ein Sauerstoffatom oder eine Gruppe NH,
R³ steht für ein Wasserstoffatom oder Methyl.

6. Pulverförmiges kosmetisches Mittel nach einem der vorstehenden Ansprüche, **dadurch gekennzeichnet, dass** das festigende Polymer der Komponente (b) bezogen auf das Gesamtgewicht der Zusammensetzung in einer Menge von 2,0 Gew.-% bis 95 Gew.-%, insbesondere von 5,0 Gew.-% bis 95,0 Gew.-%, ganz besonders bevorzugt von 7,5 bis 95,0 Gew.-%, enthalten ist.

7. Pulverförmiges kosmetisches Mittel nach einem der vorstehenden Ansprüche, **dadurch gekennzeichnet, dass** es bezogen auf sein Gewicht nicht mehr als 8 Gew.-%, insbesondere nicht mehr als 5 Gew.-%, einer Flüssigkeit enthält.

8. Pulverförmiges kosmetisches Mittel nach Anspruch nach einem der vorstehenden Ansprüche , **dadurch gekennzeichnet, dass** der volumenbezogene mittlere Partikeldurchmesser (d0,5) des Pulvers in einem Bereich von 10 µm bis 800 µm, insbesondere von 25 µm bis 500 µm, liegt.

9. Verfahren zur kosmetischen Behandlung menschlicher Haare, in dem ein kosmetisches Mittel nach einem der Ansprüche 1 bis 8 auf gegebenenfalls feuchtes Haar aufgetragen wird und auf dem Haar belassen wird.

10. Verfahren zur kosmetischen Behandlung menschlicher Haare nach Anspruch 9, in dem
(i) die Haare angefeuchtet werden,
(ii) auf die feuchten Haare ein pulverförmiges kosmetisches Mittel, nach einem der Ansprüche 1 bis 8 aufgetragen wird,
(iii) die Haare mechanisch beansprucht werden,
(iv) die Haare getrocknet werden,
mit der Maßgabe, dass die Haare nach Schritt (ii) nicht gespült werden.

11. Verfahren zur kosmetischen Behandlung menschlicher Haare nach Anspruch 9, in dem
(i) eine als Applikationshilfe genutzte Oberfläche befeuchtet wird,
(ii) ein pulverförmiges kosmetisches Mittel, nach einem der Ansprüche 1 bis 8 auf die befeuchtete Oberfläche der Applikationshilfe aufgetragen wird,
(iii) die Haare mit der Oberfläche aus (ii) zur Applikation des angefeuchteten, besagten kosmetischen Mittels in Kontakt gebracht und zur Frisur gelegt werden,
(iv) die Haare getrocknet werden,
mit der Maßgabe, dass die Haare nach Schritt (iii) nicht gespült werden.

12. Verfahren nach Anspruch 11, **dadurch gekennzeichnet, dass** die als Applikationshilfe genutzte Oberfläche die Handinnenfläche ist.

13. Verwendung eines pulverförmigen kosmetischen Mittels gemäß eines der Ansprüche 1 bis 8 zur temporären Verformung keratinhaltiger Fasern, insbesondere menschlicher Haare.

## Claims

1. A powdered cosmetic agent, containing at least one polymer as component (a), comprising at least one structural unit of formula (I) and at least one structural unit of formula (II), 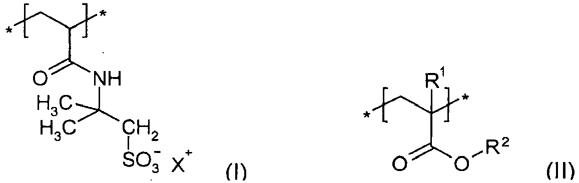
wherein X⁺ represents a physiologically compatible cation (in particular Na⁺),
R¹ represents a hydrogen atom or a methyl group, and
R² represents a (C₂ to C₆) hydroxyalkyl group,
and containing at least one stabilizing polymer as component (b).

2. The powdered cosmetic agent according to claim 1, **characterized in that** R² represents a 2-hydroxyethyl group.

3. The powdered cosmetic agent according to one of the preceding claims, **characterized in that** the polymer of component (a), based on the total weight of the composition, is contained in an amount of from 5 to 95 wt.%, particularly preferably of from 5.0 to 40.0 wt.%, and very particularly preferably of from 10.0 to 30.0 wt.%.

4. The powdered cosmetic agent according to one of the preceding claims, **characterized in that** the stabilizing polymer of component (b) is selected from polymers comprising at least one lactam structural unit.

5. The powdered cosmetic agent according to one of the preceding claims, **characterized in that** the stabilizing polymer of component (b) comprises at least one structural unit of formula (III) and at least one structural unit of formula (IV), 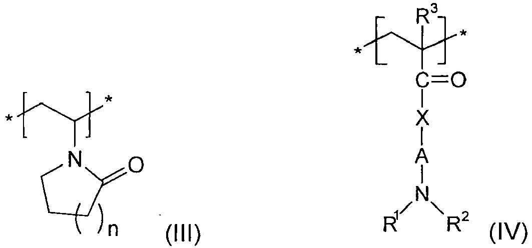
n represents 1, 2 or 3,
R¹ and R² independently represent a (C₁ to C₆) alkyl group (in particular methyl),
A represents ethane-1,2-diyl or propane-1,3-diyl,
X represents an oxygen atom or an NH group,
R³ represents an oxygen atom or methyl.

6. The powdered cosmetic agent according to one of the preceding claims, **characterized in that** the stabilizing polymer of component (b), based on the total weight of the composition, is contained in an amount of from 2.0 wt.% to 95 wt.%, in particular of from 5.0 wt.% to 95.0 wt.%, very particularly preferably of from 7.5 to 95.0 wt.%.

7. The powdered cosmetic agent according to one of the preceding claims, **characterized in that**, based on its weight, it contains no more than 8 wt.%, in particular no more than 5 wt.%, of a liquid.

8. The powdered cosmetic agent according to one of the preceding claims, **characterized in that** the average particle diameter (d0.5) by volume of the powder is in a range of from 10 µm to 800 µm, in particular of from 25 µm to 500 µm.

9. A method for cosmetically treating human hair, in which a cosmetic agent according to one of claims 1 to 8 is applied to optionally wet hair and left on the hair.

10. The method for cosmetically treating human according to claim 9, in which
(i) the hair is wetted,
(ii) a powdered cosmetic agent according to one of claims 1 to 8 is applied to the wet hair,
(iii) the hair is mechanically stressed,
(iv) the hair is dried,
with the proviso that the hair is not rinsed after step (ii).

11. The method for cosmetically treating human hair according to claim 9, in which
(i) a surface used as an application aid is wetted,
(ii) a powdered cosmetic means according to one of claims 1 to 8 is applied to the wetted surface of the application aid,
(iii) the hair is brought into contact with the surface from (ii) in order to apply the wetted, aforesaid cosmetic agent, and then styled,
(iv) the hair is dried,
with the proviso that the hair is not rinsed after step (iii).

12. The method according to claim 11, **characterized in that** the surface used as an application aid is the palm of the hand.

13. The use of a powdered cosmetic agent according to one of claims 1 to 8 for temporarily deforming keratin-containing fibers, in particular human hair.

## Revendications

1. Produit cosmétique sous forme de poudre, contenant en tant que composant (a) au moins un polymère, comprenant au moins un motif structurel de la formule (I) et au moins un motif structurel de la formule (II), 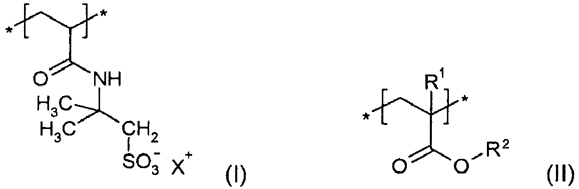
X⁺ représentant un cation physiologiquement compatible (notamment Na⁺),
R¹ représentant un atome d'hydrogène ou un groupe méthyle et
R² représentant un groupe hydroxyalkyle (C₂ à C₆),
et en tant que composant (b) au moins un polymère fortifiant.

2. Produit cosmétique en poudre selon la revendication 1, **caractérisé en ce que** R² représente un groupe 2-hydroxyéthyle.

3. Produit cosmétique en poudre selon l'une quelconque des revendications précédentes, **caractérisé en ce que** le polymère du composant (a), rapporté au poids total de la composition, est présent dans une quantité de 5 à 95 % en poids, très préférablement de 5,0 à 40,0 % en poids et on ne peut plus préférablement de 10,0 à 30,0 % en poids.

4. Produit cosmétique en poudre selon l'une quelconque des revendications précédentes, **caractérisé en ce que** le polymère fortifiant du composant (b) est sélectionné parmi des polymères comprenant au moins un motif structurel de lactame.

5. Produit cosmétique en poudre selon l'une quelconque des revendications précédentes, **caractérisé en ce que** le polymère fortifiant du composant (b) comprend au moins un motif structurel de la formule (III) et au moins un motif structurel de la formule (IV), 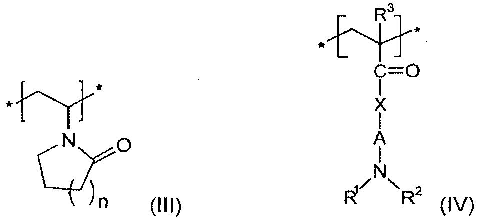
n représente 1, 2 ou 3
R¹ et R² représentent indépendamment l'un de l'autre un groupe alkyle (C₁ à C₆) (notamment du méthyle)
A représente de l'éthane-1,2-diyle ou du propane-1,3-diyle,
X représente un atome d'oxygène ou un groupe NH,
R³ représente un atome d'hydrogène ou du méthyle.

6. Produit cosmétique selon l'une quelconque des revendications précédentes, **caractérisé en ce que** le polymère fortifiant du composant (b), rapporté au poids total de la composition, est présent dans une quantité de 2,0 % en poids à 95 % en poids, notamment de 5,0 % en poids à 95,0 % en poids, on ne peut plus préférablement de 7,5 à 95,0 % en poids.

7. Produit cosmétique selon l'une quelconque des revendications précédentes, **caractérisé en ce qu'**il contient, rapporté à son poids, au maximum 8 % en poids, notamment au maximum 5 % en poids d'un liquide.

8. Produit cosmétique selon l'une quelconque des revendications précédentes, **caractérisé en ce que**, rapporté au volume, le diamètre moyen de particule (d0,5) de la poudre se trouve dans une plage allant de 10 µm à 800 µm, notamment de 25 µm à 500 µm.

9. Procédé de traitement cosmétique des cheveux humains, grâce auquel un produit cosmétique selon l'une quelconque des revendications 1 à 8 est appliqué sur des cheveux humides le cas échéant et est maintenu sur le cheveu.

10. Procédé de traitement cosmétique des cheveux humains selon la revendication 9, grâce auquel
(i) les cheveux sont humidifiés,
(ii) un produit cosmétique en poudre selon l'une quelconque des revendications 1 à 8 est appliqué sur les cheveux humides,
(iii) les cheveux sont sollicités mécaniquement,
(iv) les cheveux sont séchés,
à la condition que les cheveux ne soient pas rincés après l'étape (ii).

11. Procédé de traitement cosmétique des cheveux humains selon la revendication 9, grâce auquel
(i) une surface utilisée en tant qu'auxiliaire d'application est humidifiée,
(ii) un produit cosmétique en poudre selon l'une quelconque des revendications 1 à 8 est appliqué sur la surface humidifiée de l'auxiliaire d'application,
(iii) les cheveux sont mis en contact avec la surface à partir de (ii) pour l'application du produit cosmétique humidifié énoncé,
(iv) les cheveux sont séchés,
à la condition que les cheveux ne soient pas rincés après l'étape (iii).

12. Procédé selon la revendication 11, **caractérisé en ce que** la surface utilisée comme auxiliaire d'application est la paume de la main.

13. Utilisation d'un produit cosmétique en poudre selon l'une quelconque des revendications 1 à 8 pour la déformation temporaire de fibres à base de kératine, notamment les cheveux humains.
